Europäisches Patentamt

European Patent Office

Office européen des brevets

(19)

(11) Numéro de publication: **0 077 244**
**B1**

(12) # FASCICULE DE BREVET EUROPEEN

(45) Date de publication du fascicule du brevet:
04.09.85

(51) Int. Cl.⁴: **A 61 K 9/20**, A 23 P 1/00

(21) Numéro de dépôt: **82401777.6**

(22) Date de dépôt: **30.09.82**

(54) **Procédé de fabrication de tablettes à base de protéine.**

(30) Priorité: **09.10.81 FR 8119097**

(43) Date de publication de la demande:
**20.04.83 Bulletin 83/16**

(45) Mention de la délivrance du brevet:
**04.09.85 Bulletin 85/36**

(84) Etats contractants désignés:
**AT BE CH DE GB IT LI LU NL SE**

(56) Documents cités:
**FR - A - 2 397 792**
**GB - A - 1 538 280**

(73) Titulaire: **Laboratoires MACORS S.A., 20 Rue des Fabriques, F-77000 Melun (FR)**
Titulaire: **Société Civile d'Inventeurs C.L.P., 41 Rue Camille Pelletan, F-92305 Levallois (FR)**

(72) Inventeur: **Dupontreue, Jacques, 21 Avenue d'Iéna, F-75016 Paris (FR)**
Inventeur: **Pignon, Roger, Tour Eve, Appartement 2002 1 Place du Sud, F-92800 Puteaux (FR)**

(74) Mandataire: **Lemonnier, André, 4 Boulevard Saint-Denis, F-75010 Paris (FR)**

ACTORUM AG

## Description

La présente invention concerne la fabrication des tablettes à base de protides, et notamment de protéines, à usage diététique, alimentaire ou pharmaceutique.

Actuellement des comprimés sont obtenus par mélange à sec de protéines, d'adjuvants, de produits actifs et d'additifs autorisés et par compression mécanique, à l'aide d'une machine alternative ou rotative, de doses du mélange.

Ces produits ont une mauvaise tenue mécanique et sont très secs au goût.

La présente invention a pour but de fabriquer des tablettes à base de protéine présentant une bonne acceptabilité gustative mais surtout présentant une très bonne tenue mécanique, ce qui permet entre autres de les utiliser en diététique et notamment dans les régimes hypocaloriques ou comme véhicules pharmaceutiques.

Le procédé de fabrication de tablettes à base de protéine conforme à l'invention est caractérisé par la combinaison des stades suivants:

a) mouillage de la protéine avec de l'eau sous pression;

b) granulation de la protéine ainsi mouillée, séchage et calibrage des granulés;

c) enrobage des granulés avec un lipide, et

d) moulage par compression des grains enrobés avec maintien de la pression.

Dans le procédé, les adjuvants, les produits actifs et les additifs autorisés que l'on désire incorporer aux tablettes sont ajoutés aux granulés de protéine après l'enrobage avec le lipide et avant le moulage par compression.

On expliquera et détaillera ci-après les différents stades du procédé en prenant pour exemple la fabrication de 130 kg de tablettes diététiques à faible apport calorique.

On a mouillé 100 kg de protéine de lait avec de l'eau sous une pression de 200 kPa. La quantité d'eau mise en œuvre était de 22 litres. Contrairement à ce qui était admis, le mouillage de la protéine a été parfaitement réalisé sous une pression de 200 kPa.

La protéine mouillée a été déversée dans un mélangeur granulateur et granulée sous forme de grains de 2 à 3 mm de diamètre. Ces grains ont été séchés à l'étuve ventilée entre 45 et 50°C pendant 8 heures et calibrés dans un granulateur sur une grille de 2 mm d'ouverture de maille.

Les grains calibrés ont alors été enrobés avec un lipide d'origine palmique. La quantité de lipide mise en œuvre était égale à environ 9% en poids des granulés. Les granulés se sont ainsi trouvés enrobés avec un lipide réparti à la surface des granulés qui, de ce fait, allait se trouver parfaitement dispersé de façon homogène dans la masse. Ce lipide, d'une part, améliore la cohésion après compression et, d'autre part, donne un moelleux qui améliore les propriétés gustatives.

A ce stade on a ajouté aux grains enrobés, dans le mélangeur granulateur, environ 20 kg de glucides sous forme de fructose et de miel et environ 3% de cellulose microcristalline et ces additifs, au cours du processus de mélange, ont enrobé les grains déjà enrobés par le lipide. La cellulose microcristalline a essentiellement pour but d'accroître la fluidité des granulés et de faciliter le remplissage des moules. Au cours de ce stade il serait possible d'incorporer, au lieu des glucides ou simultanément avec ceux-ci, d'autres adjuvants, des produits pharmaceutiquement actifs, des additifs autorisés, etc.

Les granulés ont alors été répartis dans les empreintes d'un moule à raison de 7 g par empreinte et la compression a été effectuée sous 25 000 kPa. Dans ce stade il est, d'une manière surprenante, essentiel de maintenir la pression pendant au moins 5 secondes. Des essais de compression sur les presses alternatives ou rotatives utilisées habituellement dans l'industrie pharmaceutique dans lesquelles la pression maximale n'est atteinte que pendant le passage d'un point mort, ont donné des tablettes feuilletées sans cohésion mécanique. Par contre la compression effectuée sur une presse hydraulique ou avec tout autre type de presse autorisant le maintien de la pression, donne des tablettes présentant une bonne cohésion et une bonne tenue mécanique.

Les tablettes de 7 g obtenues comportaient 4,20 g de protides, 0,60 g de lipides et 1,40 g de glucides avec une valeur énergétique de 117 kJ.

## Revendications

1. Un procédé de fabrication de tablettes à base de protéine, caractérisé par la combinaison des stades suivants:

a) mouillage de la protéine avec de l'eau sous pression;

b) granulation de la protéine ainsi mouillée, séchage et calibrage des granulés;

c) enrobage avec un lipide des granulés, et

d) moulage par compression des grains enrobés avec maintien de la pression.

2. Un procédé de fabrication de tablettes à base de protéine selon la revendication 1, caractérisé en ce que les adjuvants, les produits actifs et les additifs autorisés sont ajoutés aux granulés de protéine après l'enrobage avec le lipide et avant le moulage par compression.

3. Un procédé de fabrication de tablettes à base de protéine selon l'une quelconque des revendications 1 ou 2, caractérisé en ce que le mouillage de la protéine est effectué sous une pression de 200 kPa.

4. Un procédé de fabrication de tablettes à base de protéine selon la revendication 3, caractérisé en ce que la quantité d'eau utilisée pour le mouillage est de 22% en poids par rapport au poids de la protéine.

5. Un procédé de fabrication de tablettes à base de protéine selon l'une quelconque des revendications 1 à 4, caractérisé en ce que la protéine mouillée est granulée sous forme de grains passant au tamis de 2 mm d'ouverture de maille.

6. Un procédé de fabrication de tablettes à base de protéine selon la revendication 1, caractérisé en ce que le lipide utilisé pour l'enrobage est mis en œuvre à raison de 9% en poids du poids des granulés.

7. Un procédé de fabrication de tablettes à base de protéine selon l'une quelconque des revendications 1 à 6, caractérisé en ce que le moulage par compression est effectué sous pression d'environ 25 000 kPa maintenue pendant au moins 5 secondes.

## Patentansprüche

1. Verfahren zur Herstellung von Tabletten auf der Grundlage von Protein, gekennzeichnet durch die Kombination folgender Verfahrenschritte:

a) Befeuchtung des Proteins mit Wasser unter Druck;

b) Granulation des so befeuchteten Proteins, Trocknung und Kalibrierung der Teilchen;

c) Ummantelung der Teilchen mit einem Lipid und

d) Pressformung der ummantelten Teilchen mit Aufrechterhaltung des Druckes.

2. Verfahren zur Herstellung von Tabletten auf der Grundlage von Protein nach Anspruch 1, dadurch gekennzeichnet, dass die Adjuvantia, die Wirkstoffe und die zugelassenen Hilfsmittel den Protein-Teilchen nach der Ummantelung mit dem Lipid und vor der Pressfurmung hinzugefügt werden.

3. Verfahren zur Herstellung von Tabletten auf der Grundlage von Protein nach einem der Ansprüche 1 oder 2, dadurch gekennzeichnet, dass die Befeuchtung des Proteins unter einem Druck von 200 kPa durchgeführt wird.

4. Verfahren zur Herstellung von Tabletten auf der Grundlage von Protein nach Anspruch 3, dadurch gekennzeichnet, dass die Menge des für die Befeuchtung verwendeten Wassers 22 Gew.-% des Gewichts des Proteins beträgt.

5. Verfahren zur Herstellung von Tabletten auf der Grundlage von Protein nach einem der Ansprüche 1 bis 4, dadurch gekennzeichnet, dass das befeuchtete Protein in Teilchen granuliert wird, indem es durch ein Sieb mit einer Maschenweite von 2 mm gegeben wird.

6. Verfahren zur Herstellung von Tabletten auf der Grundlage von Protein nach Anspruch 1, dadurch gekennzeichnet, dass das für die Ummantelung verwendete Lipid in der Menge von 9 Gew.-% des Gewichts des Granulats zum Einsatz kommt.

7. Verfahren zur Herstellung von Tabletten auf der Grundlage von Protein nach einem der Ansprüche 1 bis 6, dadurch gekennzeichnet, dass die Pressformung unter einem Druck von ca. 25 000 kPa erfolgt, und dass dieser Druck mindestens 5 Sekunden aufrechterhaltung wird.

## Claims

1. A method for manufacturing protein containing tablets, characterized by the combination of the following steps:

a) hydrating protein with pressurized water;

b) granulation of the so hydrated protein, drying and calibrating of the granulates;

c) coating of the granulates with a lipid, and

d) compression moulding of the coated grains with maintaining the pressure.

2. A method for manufacturing protein containing tablets according to claim 1, characterized in that the adjuvants, the active products and the authorized additives are added to the protein granulates after coating with the lipid and before the compression moulding.

3. A method for manufacturing protein containing tablets according to any of the claim 1 or 2, characterized in that hydratating of the protein is made under a pressure of 200 kPa.

4. A method for manufacturing protein containing tablets, according to claim 3, characterized in that the amount of water used for hydrating is by weight equal to 22% of the weight of the protein.

5. A method for manufactured protein containing tablets according to any of claims 1 to 4, characterized in that the hydrated protein is granulated in the form of 2 mm passing pellets.

6. A method for manufacturing protein containing tablets according to claim 1, characterized in that the lipid used for the coating amounts to 9% of the weight of the pellets.

7. A method for manufactured protein containing tablets according to any of claims 1 to 6, characterized in that the compression moulding is achieved under a pressure of ca 25 000 kPa, wich is maintained during at least 5 seconds.